# EUROPEAN PATENT SPECIFICATION

(11) **EP 4 048 217 B1**
(45) Date of publication and mention of the grant of the patent: **11.06.2025**
(21) Application number: 19845733.5
(22) Date of filing: 05.12.2019
(51) Int. Cl.: A61F 13/20

(54) **TEARING AND WINDING ASSEMBLY IN A TAMPON MANUFACTURING APPARATUS AND METHOD FOR MANUFACTURING SUCH TAMPON BY MEANS OF SUCH APPARATUS**
ABREISS- UND AUFWICKELVORRICHTUNG IN EINER TAMPONHERSTELLUNGSVORRICHTUNG UND VERFAHREN ZUR HERSTELLUNG EINES SOLCHEN TAMPONS MITTELS EINER SOLCHEN VORRICHTUNG
ENSEMBLE DE DÉCHIREMENT ET D'ENROULEMENT DANS UN APPAREIL DE FABRICATION DE TAMPONS ET PROCÉDÉ DE FABRICATION DESDITS TAMPONS AU MOYEN DUDIT APPAREIL

(30) Priority: 13.08.2019 SI 201900143
(43) Date of publication of application: 31.08.2022
(73) Proprietor: TOSAMA Tovarna sanitetnega materiala d.o.o., 1230 Domzale (SI)
(72) Inventor: JERIN, Franci, 1225 Lukovica (SI)
(74) Representative: Borstar, Dusan
(86) International application number: PCT/SI2019/000008
(87) International publication number: WO 2021/029829

(56) References cited:
- EP-A1- 3 412 264

## Description

The invention substantially refers to a an apparatus and a method for manufacturing of a tampon, in particular a hygienic tampon, which is made of a band consisting of absorbent fibers, wherein said band is together with a liquid permeable wrapper, which is placed thereon, and a retaining string, which is placed around both of them, wound around its transversal axis wound, by which an essentially cylindrical blank is obtained, which is then formed and finalized by radial compression to a pre-determined density, wherein optionally also a proximal portion, namely a so-called tampon head is formed, which is suitable for insertion of the tampon, so that such tampon may then be used either as a digital or applicator tampon. The invention in particular refers to a tearing-off assembly, by means of which said section of said band, which is wound into each blank, is separated from the residual endless band, and moreover also to method for manufacturing such tampon. Pursuant to the International Patent Classification such inventions in the field of common life necessities belong to medicine and hygiene, namely to tampons, in particular to menstrual hygienic tampons, which are made of wound material and therefore belongs to class A 61 F 13/22.

The purpose of the invention is to create an apparatus as well as a method for manufacturing of a tampon on the basis of a suitably wound longitudinal section of a band consisting of absorbent fibers, which is upon said winding of said section of the band exposed to radial compression to each desired density, wherein a undesirable oval shape of the cross-section of the wound section prior to compression due to suddenly and step-like cutting of the terminal edge of the band should be omitted, and the band shall not only be suitable for fluent transferring thereof through the apparatus, but should also be ended smoothly and gradually by gradually reducing the density of fibers in the longitudinal direction of the band, which is easily achievable by tearing and leads to essential dissipation of the length and distribution of absorbent fibers in the area of separating each section of the band apart from the rest of the endless band, so that upon winding of such torn band the shape of each obtained blank is essentially cylindrical already before the radial compression, which then consequently during the use of such finalized tampon, namely by exposing thereof to moisture and absorption of each liquid, which the tampon is capable to absorb and retain, leads to essentially more controlled and uniform expansion of the tampon along its complete cross-section.

A tampon, which is disclosed in EP 0 422 660, consists of a nonwoven fibrous blank, which is formed by winding of a section of a nonwoven fibrous band around its transversal axis, by which at least approximately cylindrical blank is obtained, which is then transformed to a tampon by compression in its radial direction by means of suitable jaws and a suitable apparatus like e.g. those as disclosed in DE 19 825 877. Prior to that, at least one additional layer consisting of a liquid permeable material, a so-called wrapper, is placed above the previously mentioned band, and moreover, both of said components are surrounded by a retaining string, which is during the subsequent winding anchored within the blank, while a part of said string remains located outside of the blank and can be later used as a requisite, by which the tampon after the use can be removed e.g. from a body cavity. Such tampon should be available in a cylindrical shape and is on its surface furnished with radially inward impressed grooves, which extend in the longitudinal direction of the tampon i.e. parallel with the central longitudinal axis of the tampon. Such tampons are generally known as digital tampons. A rib is available between each two neighboring grooves, and the peripheral circumference of said ribs defines each diameter of a tampon, while the bottom areas of said grooves define a diameter of an essentially compressed core. By performing of an additional and relatively slight radial compression in the area of said ribs it is then achieved that the lateral surfaces of each ribs are convex i.e. deflected, so that the lateral surfaces of each neighboring ribs are brought in contact with each other and therefore form a tubular space adjacent to said compressed core, which extends parallel to said longitudinal central axis of the tampon. Such concept should result in several beneficial effects, among others also in increasing of buckling and bending strength of a tampon, and in particular also to increasing of specific absorptiveness and absorption rate in the longitudinal direction of the tampon. Stability of a tampon is crucial in the sense of entrustment from the side of each user and is in particular important by inserting of a tampon. In the view of inserting, in addition to said stability it is also quite important that the external surface of a tampon, which is during the use permanently in contact with a mucosa, is made as smooth as possible, which is achieved by placing said wrapper at least onto the external areas of the ribs on said tampon. The purpose of using such wrapper is disclosed in EP 1 035 819 or US 2003/0105444 A1, by which already in the stage of winding of a layer of natural fibrous material into an at least approximately cylindrical blank, which is then by radial compression performed by suitable jaws transformed into a tampon, a previously smoothed wrapper is placed over said fibrous layer, and is then together with said fibrous layer wound into a blank. As a result of subsequent radial compression by means of said radially extending jaws, a tampon is obtained, which is along its complete circumference surrounded by said wrapper.

One of the problems, to which the users and also the manufacturers are focused in the field of such known tampons, is the fact that already during the production the actual shape of said blank is not really cylindrical and its cross-section is not really circular, but is shaped as a double helical band, the terminal portions of which are step-like ended. Due to the thickness of said helically wound band the discrepancy between the ideal circular profile and actual profile of the blank in the area of said terminal portions thereof is essential, and said end portions are no doubt disturbing. On the one hand, such straightforwardly and step-like cut terminal portion has always a tendency of unwinding or folding, and on the other hand it is also problematic by performing an uniform radial compression by means of usually eight jaws, which are equidistantly apart from each other arranged along the circumference of the blank. A really uniform compression can actually be assured only in the case, if the transversal cross-section is really round. As there is always a discrepancy between the ideal circular cross-section and the actual cross-section of a helically wound band, after compression the fibers are not uniformly distributed along the complete cross-section, so that upon compression also the shape of such obtained tampon is not perfectly cylindrical. This is actually not so much obvious and problematic prior to use of a tampon, but the problem arises later-on during the use, when such tampon is exposed to moisture and is used to absorb each liquid, which is present in particular the area adjacent to its proximal end portion and/or its circumferential surface. But when such tampon is wet, its volume is increased, and due to such irregular distribution and density of fibers and consequently also varying of absorptiveness its shape may also be essentially changed.

Tampons are known in the state of the art, by which said terminal portion of the band of absorptive fibers is not step-like cut, and said band is prior to compression ended gradually. Such gradually ended terminal portion is actually not cut but is torn-off, so that the density of fibers in both terminal areas of the band is gradually reduced, and longer fibers as available in the area of such torn terminal portion are then simply wound around the blank. Consequently, a blank is obtained, the cross-section of which is still shaped as a helically wound band having a pre-determined thickness, but its terminal portions in the circumferential area of the blank are pretty smooth and in particular not step-like or protruding outwardly, so that the silhouette of such blank is much closer to the circle than by a blank made of straightforwardly and step-like cur band.

Method for manufacturing such tampon in accordance with the previously discussed approach, in which the band is not cut but rather torn, and also a tearing assembly in an apparatus, which is suitable for manufacturing such tampon, are both disclosed in EP 3 412 264 A1.

A process of tearing-off and winding of said absorbent band in accordance with said EP 3 412 264 A1 comprises
- providing of displacing of an endless absorbent fibrous band in a processing direction;
- optionally providing of weak lines onto said band, which extend along the transversal cross-section thereof;
- pulling of said band by means of friction by the first linear velocity in the area of its first operational position in the apparatus, wherein by taking into consideration each processing direction said second position is located in front of said first position;
- optionally guiding said band by means of at least one guiding means towards the third position, wherein by taking into consideration each processing direction said third position is located in front of said first and second position;
- tearing-off a section of said fibrous absorbent band apart from the endless band by means of pulling the band in said third position, wherein by taking into consideration each processing direction said third position is located in front of said first and second position, and wherein said pulling is performed by means of establishing said second linear velocity in the third position, which is higher than first linear velocity, by which a force is ensured, which is sufficient for efficient tearing-off said band in the area between said first and said third position; and
- winding of said torn-off section of the absorbent band,
wherein the absorbent band is in the first position maintained at the first linear velocity, while by pulling towards the third position its linear velocity in the second position is higher than the first linear velocity.

Said method moreover provides that the band of absorbent fibers is in the third position pulled by means of its engagement in a rotational clamping unit, and is rotated by a velocity, which allows establishing of said second linear velocity of displacing the belt. Said method also provides that a non-woven layer i.e. a wrapper is prepared, onto which in said first position an absorbent fibrous band is placed, upon which a string is placed around both of them, wherein by winding of the absorbent band said wrapper is located on the external side and the free terminal portion of the string protrudes outside from the area of said band and said wrapper.

Said method further provides that a step is included, in which each displacing of the absorbent band is limited in a direction perpendicularly with regard to the band, namely in the area between the first and the second position and in the area between the second and the third position. Preventing of hindering by winding the band is also foreseen.

Moreover, an apparatus for tearing and winding of an absorbent fibrous band during manufacturing of a tampon is also disclosed in the previously mentioned EP 3 412 264 A1. Such apparatus comprises a first pair of rollers, a second pair of rollers and a pulling means, and the apparatus enables displacing said endless band from said first pair of rollers towards the second pair of rollers and then from said second pair of rollers towards said pulling means, wherein said first pair of rollers is embedded within a first frame and the distance between the rollers is smaller than the thickness of the band, while the second pair of rollers is embedded within a second frame and the distance between the rollers is also smaller than the thickness of the band, and wherein for the purpose of engagement and displacing of said band it is foreseen that both rollers in the first pair are able to rotate and to displace said band by friction and with a first linear speed, while both rollers in the second pair are also able to rotate and also to displace said band by friction with a linear speed, which corresponds to at least first linear speed, and wherein said pulling means is suitable for periodically pulling said band with a second linear speed, which is higher than said first linear speed and therefore enables tearing-off a section of a band from the residual endless band. In this, said first pair of rollers should rotate by a first linear speed and the second pair of rollers is acting on the principle of a fly wheel and should therefore by transmission of said band there-between rotate with a speed, which is higher than said first linear speed.

It is also foreseen in such apparatus that said pulling means includes a rotatable engagement means, which is usually annotated as a fork. Said pulling means also comprises a casing, by which said rotatable engagement means is surrounded and which is furnished with recesses, through which said absorbent band is inserted, while said casing is serving for spatial limitation of each wound blank when prepared for the purpose of manufacturing a tampon.

As further foreseen in such apparatus, a guiding means comprises at least one pair of optionally displaceable guiding sheets, and each of said guiding sheets includes a recess, through which said absorbent band is inserted, by which each movement of said absorbent band transversally with respect to a direction of progressing the band, wherein at least one pair of said guiding sheets is available between said first position and said second position, and at least one pair of said guiding sheets is available between said second position and said third position.

As still further foreseen, such apparatus shall also comprise a means, which is suitable for making a weak line on said absorbent band prior to insertion thereof between the first pair of rollers, and said means should comprise a blade.

As still further foreseen, such apparatus shall also comprise a means for tearing-off a section of the absorbent band apart from the residual endless band, a means for attaching said wrapper onto said absorbent band, a means for placing said string around said absorbent band and said wrapper, and a means for cutting said string.

For the purpose of guiding of said absorbent band in each processing direction the band must be permanently guided, since otherwise it could start running in irregular direction. Besides, said second pair of rollers is functioning as a fly wheel, which means that the rollers are pretty heavy and is therefore not easy to stop them upon tearing, so that an additional breaking means is required, without which the free terminal portion of the endless band would continue moving towards the second pair of rollers and unwinding of the endless band would be continued even upon the tampon manufacturing apparatus is switched-off. In addition to that, it is also clear for each person skilled in the art that due to such concept of the apparatus and also the method as disclosed, performing of said weak line extending transversally with respect to each processing direction appears to be unavoidable, since without this measure each properly defined tearing of the band could not be performed in such way, that winding of the band and performing of each further steps in course of finalizing the tampon could be possible.

The present invention refers to a tearing and winding assembly in a tampon manufacturing apparatus, especially for manufacturing of a hygienic tampon. Such tampon consists of an endless band, which is formed of absorbent fibers and cut to longitudinal sections of a pre-determined length, and which is together with a liquid permeable wrapper and a retaining string helically wound into at least approximately cylindrical blank, which is then radially compressed into at least approximately cylindrical tampon, which is on its circumference furnished with a plurality of longitudinally extending ribs, which are along the circumference equidistantly spaced apart from each other.

Said tearing assembly in said tampon manufacturing apparatus comprises two pairs of guiding rollers. The rollers in the first pair are arranged at a pre-determined distance apart from each other, which is smaller than the thickness of said band, and are rotatably embedded in a casing, and are moreover pressed towards to each other with a pre-determined force and rotated in opposite directions with a pre-determined circumferential velocity and are therefore suitable for guiding said band there-between in a processing direction, namely in a direction towards the second pair of rollers. On the other hand also the rollers in said second pair are arranged at a pre-determined distance apart from each other, which is smaller than thickness of said band, and are rotatably embedded in a suitable casing and moreover also pressed towards to each other by a pre-determined force and simultaneously rotated in opposite directions with a circumferential velocity, and are therefore also suitable for guiding said band there-between in a processing direction, namely towards a winding assembly in a tampon manufacturing apparatus.

Said winding assembly comprises a pre-determined number of winding units, which are arranged equidistantly along the circumference of a virtual cylinder, wherein each winding unit comprises a bifurcated rotor, which is coaxially inserted into a tubular stator. Said rotor is furnished with a diametrically extending throughout passage, which is suitable for receiving said longitudinal section of the band. Said stator is furnished with a diametrically extending passage, which is suitable for receiving of the same previously mentioned longitudinal section of the band. Consequently, as soon as each longitudinal section of the band is inserted both through said passage in the stator and said passage in the rotor, and upon that by performing a pre-determined number of revolutions of the rotor, said winding unit is suitable for formation of a helically wound section of the absorbent band together with said wrapper and said retaining string into an at least approximately cylindrical blank, which is ready to be radially compressed into a properly shaped and finalized tampon, and wherein in front of said tearing assembly when taking in consideration said processing direction.

A further assembly can be optionally available, which is intended for periodical weakening of the band prior to tearing, by which the length of longitudinal sections is defined, to which the band is then cut.

The present invention proposes that said tearing assembly comprises a stationary first pair of rollers, which are by circumferential velocity rotated in opposite directions, as well as an oscillatory to and fro along the plane of transferring said absorbent band displaceable second pair of rollers, which are also rotated in opposite directions by constant circumferential velocity. Thanks to said oscillatory displacing, said second pair of rollers is during uniformly and continuously transferring said band between said pairs of rollers by a velocity, which at least approximately corresponds to the circumferential velocity of the first pair of rollers, in periodically repeating manner displaceable from its first terminal position, which is located adjacent to said first pair of rollers, to its second terminal position, which is located in said plane of transferring said sections of the band at a pre-determined distance apart from said first pair of rollers, and vice versa. By oscillating said second pair of oppositely rotating rollers the circumferential velocity of them remains unchangeable, while the distance between said first terminal position and said second terminal position of the second pair of rollers is determined in such manner that during displacing of the second pair of rollers together with each particular section of the absorbent band as jammed there-between in a direction away from the first pair of rollers said absorbent band is just partially torn, upon which said partially torn band is introduced into said winding assembly, namely through the passage in the rotor as well as through the passage in the stator of each disposable winding unit. As a consequence, said band is completely torn by means of rotating said rotor of the winding assembly during the reverse displacement of the second pair of rollers towards the first pair of rollers.

In one of possible embodiments of the invention said circumferential velocity of rotating of the second pair of rollers in the opposite directions at least approximately equals to the circumferential velocity of rotating of the first pair of rollers in the opposite directions, by which each particular longitudinal section of the absorbent band is transferred towards the winding assembly along a corresponding plane.

In an alternative embodiment of the invention the circumferential velocity of rotating of the second pair of rollers in the opposite directions is higher than the circumferential velocity of rotating of the first pair of rollers in the opposite directions, by which each particular longitudinal section of the absorbent band is transferred towards the winding assembly along a corresponding plane, wherein the difference between said second circumferential velocity and said first circumferential velocity is such, that during reverse displacement of the second pair of rollers from its second terminal position towards its first terminal position adjacent to the first pair of rollers each potential formation of loops on the continuously between the first pair of rollers extending band can be avoided, by which a fluent processing of said band through both pairs of rollers is assured without formation of loops or folding the band.

Both, tearing and winding assembly are optionally adapted to cooperate with a further assembly, which is suitable for providing periodical initial weakening of the band prior to its completely tearing-off, wherein said further assembly is adjusted for providing of weak zones in the form of a plurality of mutually displaced notches or grooves or passages, which are arranged in a sequence, and with regard to the processing direction of the band extend at least approximately in a transversal direction of the band. In this, said further assembly for providing said periodical weakening of the band prior to complete tearing-off comprises at least one apparatus selected from devices e suitable for cutting, notching, punching, chemical etching, water jet cutting, air jet cutting, or cutting by laser beam.

The invention also refers to a method for manufacturing of a tampon by using an apparatus comprising a tearing and winding assembly in accordance with previously disclosed features, wherein said method comprises
- providing of an endless band consisting of absorbent fibers and having a pre-determined width and at least approximately uniform thickness;
- transferring said band through an assembly, which is suitable for forming of along said band periodically repeated weak zones, which are formed either as cuttings or perforations or similar local weak zones, which are arranged in sequences extending at least approximately in the transversal direction of said band and by which the longitudinal sections of subsequently torn band are defined;
- placing of a liquid permeable layer, namely a so called tampon wrapper, over said absorbent band, and placing of a retaining string around said absorbent band;
- transferring said absorbent band through two pairs of oppositely rotating rollers due to tearing of said band and thus providing suitable longitudinal sections;
- transferring said band towards the winding assembly, in which each longitudinal section of the band in each disposable winding unit is inserted both through a passage in the rotor and the passage in the stator, by which said rotor is coaxially surrounded, upon which said rotor is rotated and upon certain number of revolutions said section of the band is helically wound into at least approximately cylindrical blank, which us then followed by at least
- compression of said blank in its radial direction by means of a pre-determined number of jaws, which includes formation of a proximal area of the tampon into a shape, which corresponds either to digital or applicator tampon, so that said blank is transformed to a tampon having a pre-determined density of fibers and consequently also each corresponding absorptiveness and mechanical strength, and is moreover furnished with a plurality of longitudinally extending ribs together with grooves there-between, which are equidistantly spaced apart of each other in the circumferential direction of such obtained tampon.

Such method according to the invention provides that said absorbent band is during its transfer throughout the tearing assembly displaced by the first velocity, which corresponds to unchangeable circumferential velocity by rotating of the first pair of rollers, between which the band is transferred, upon which said band is transferred between the second pair of rollers, the circumferential velocity of which is also unchangeable. In this, the first pair of rollers is maintained in its position, while the second pair of rollers is oscillated to and fro in a direction along the plane of transferring said band, namely from its first terminal position adjacent to the first pair of rollers to its second terminal position at a pre-determined distance apart from said first pair of rollers, and vice versa. Due to said oscillatory displacing of said second pair of rollers, said absorbent band is in the area between both pairs of rollers just partially torn, upon which such partially torn band is transferred towards each disposable winding unit in the winding assembly, namely throughout the passage in the rotor and also throughout the passage in the stator surrounding said rotor, upon which after certain number of revolutions by rotating said rotor and by simultaneously performing a reverse displacement of the second pair of rollers towards the first pair of rollers each partially torn section of said band is completely torn and separated from the rest of said endless band.

In one of variations of said method, the circumferential velocity of oppositely rotating second pair of rollers is maintained at approximately equal value as the circumferential velocity of the oppositely rotating first pair of rollers, by which then also each longitudinal section of the absorbent band is forwarded towards the winding assembly along the processing plane.

In a further variation of said method the circumferential velocity of oppositely rotating second pair of rollers is maintained higher value than the circumferential velocity of the oppositely rotating first pair of rollers, by which each longitudinal section of the absorbent band is forwarded towards the winding assembly along the processing plane, wherein the difference between said circumferential velocity of oppositely rotating second pair of rollers and the circumferential velocity of the oppositely rotating first pair of rollers is sufficient to prevent formation of a loop during the reverse movement of the second pair of rollers from its second terminal position towards its first terminal position adjacent to the first pair of rollers when the band is continuously forwarded through the first pair of rollers, by which a fluent forwarding of band through both pair of rollers is assured without forming any loops or folding of the band.

Optionally, said periodically arranged weak zones on the band are prior to final tearing performed in the form of a sequence of cutoffs or notches or throughout passages, which are spaced apart from each other and arranged in a sequence extending at least approximately transversally with regard to a direction of forwarding said band, wherein said cutoffs or notches or throughout passages are made by cutting, notching, punching, chemical etching, water jet cutting, air jet cutting, or cutting by laser beam.

The invention will be explained in more detail on the basis of an embodiment, which is presented in the attached drawings, in which
- Fig. 1: schematically presents a tearing and winding assembly in a tampon manufacturing apparatus in the first operational position thereof; while
- Fig. 2: schematically presents a tearing and winding assembly in a tampon manufacturing apparatus in the second operational position thereof.

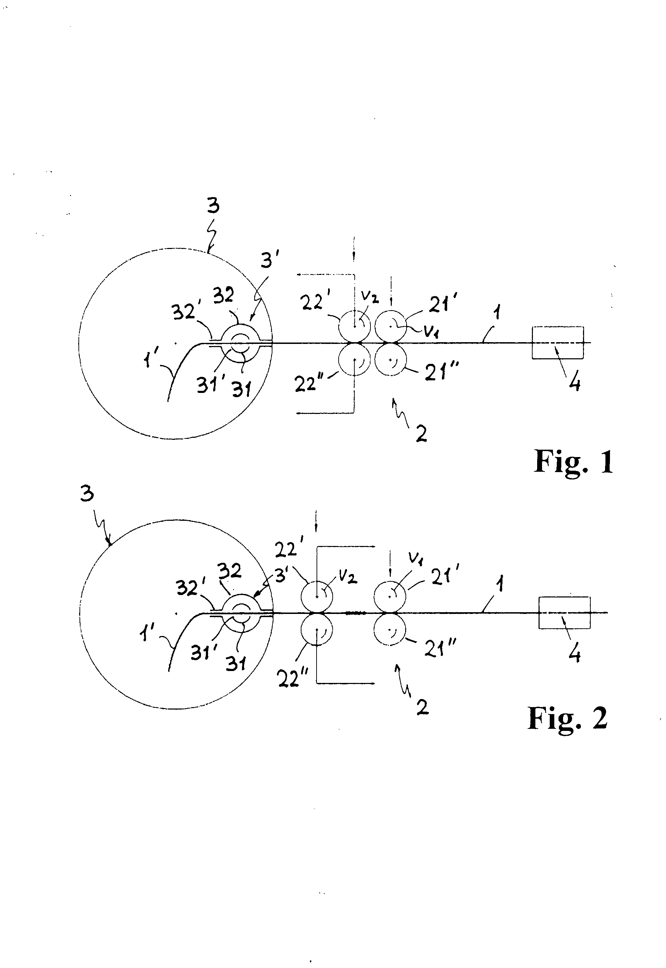

A tearing assembly 2 and a winding assembly 3 in a tampon manufacturing apparatus, especially for manufacturing of a hygienic tampon, are presented in Fig. 1, and are in the shown embodiment adapted for cooperation with a further assembly 4, which is suitable for weakening of an endless band consisting of absorbent fibers in the area of periodically repeating longitudinal sections 1'.

Both said tearing assembly 2 and said winding assembly 3 belong to a tampon manufacturing apparatus, which is suitable of manufacturing of a tampon, which consists of said longitudinal section 1' of said endless absorbent fibrous band of a pre-determined length, and is together with a liquid permeable wrapper and a retaining string helically wound into at least approximately cylindrical blank, which is then radially compressed into at least approximately cylindrical tampon, which is on its circumference furnished with a plurality of longitudinally extending ribs, which are along the circumference equidistantly spaced apart from each other. Such tampon is either a digital or applicator tampon and dimensions thereof are adjusted to standardized ones in each particular range (e.g. according to Edana). Said absorbent band 1 is an endless band having a width of 40 - 52 mm and uniform thickness both along its longitudinal and transversal direction, and its weight per length unit and in view of each expected specific absorptiveness is adjusted to 3,3 - 17,4 g/m. Such band 1 consists of absorbent plant fibers on the basis of cotton, viscose or a mixture thereof, and optionally also in combination with bamboo, hemp or nettle fibers.

It is foreseen by the manufacturing of such tampon, that separate longitudinal sections 1' are formed of said endless band 1, the length of which should be between 250 - 260 mm, measured prior to tearing-off or partial tearing thereof. Each section 1' is then on its one side, which corresponds to external side of a tampon, covered by a liquid permeable wrapper, upon which a retaining string is looped around both of them, which upon formation of each tampon remains embedded within the tampon and is intended for removing the tampon after it has been used.

Said tearing assembly 2 in said tampon manufacturing apparatus generally comprises two pairs of guiding rollers 21', 21"; 22', 22", wherein the rollers 21', 21" in the first pair are arranged at a pre-determined distance apart from each other, which is smaller than the thickness of said and (1), rotatably embedded in a casing, and are pressed towards to each other with a pre-determined force and are rotated in opposite directions with a pre-determined circumferential velocity v₁ and are therefore suitable for guiding said band 1 there-between in a processing direction, namely in a direction towards the second pair of rollers 22', 22". Also the rollers 22', 22" in said second pair are arranged at a pre-determined distance apart from each other, which is smaller than thickness of said band 1, and are rotatably embedded in a suitable casing and pressed towards to each other by a pre-determined force and are also simultaneously rotated in opposite directions with a circumferential velocity v₂, and are therefore also suitable for guiding said band 1 there-between in a processing direction, namely towards a winding assembly 3 in a tampon manufacturing apparatus. Said winding assembly as such is generally known to those skilled in the art and comprises a pre-determined number of winding units 3', which are arranged equidistantly along the circumference of a virtual cylinder, wherein each winding unit 3' comprises a bifurcated rotor 31, which is coaxially inserted into a tubular stator 32. Said rotor 31 is furnished with a diametrically extending throughout passage 31', which is suitable for receiving said longitudinal section 1' of the band. Said stator 32 is also furnished with a diametrically extending passage 32', which is suitable for receiving of the same previously mentioned longitudinal section 1' of the band 1, so that consequently, as soon as each longitudinal section 1' of the band 1 is inserted both through said passage 32' in the stator 32 and said passage 31' in the rotor 31 and upon that by performing a pre-determined number of revolutions of the rotor 31, said winding unit 3 is suitable for formation of a helically wound section 1' of the absorbent band 1 together with said wrapper and said retaining string into an at least approximately cylindrical blank, which is ready to be radially compressed into a properly shaped and finalized tampon.

In the embodiment according to Figs. 1 and 2, in front said tearing assembly 2,when taking in consideration said processing direction, a further assembly 4 is available, which is intended for periodical weakening of the band 1 prior to tearing, in which also the length of longitudinal sections 1' is defined, to which the band 1 is then cut.

In accordance with the present invention, said tearing assembly 2 comprises a stationary first pair of rollers 21', 21", which are by circumferential velocity v₁ rotated in opposite directions, as well as an oscillatory to and fro along the plane of transferring said absorbent band 1 displaceable second pair of rollers 22', 22", which are also rotated in opposite directions by constant circumferential velocity v₂. Thanks to said oscillatory displacing, said second pair of rollers 22', 22" is during uniformly and continuously transferring said band 1 between said pairs of rollers 21', 21"; 22', 22" by a velocity, which at least approximately corresponds to the circumferential velocity v₁ of the first pair of rollers 21', 21", in periodically repeating manner displaceable from its first terminal position, which is located adjacent to said first pair of rollers 21', 21", to its second terminal position, which is located in said plane of transferring said sections of the band 1 at a pre-determined distance apart from said first pair of rollers 21', 21", and vice versa. When oscillating said second pair of oppositely rotating rollers 22', 22", the circumferential velocity v₂ of them remains unchangeable, while the distance between said first terminal position and said second terminal position of the second pair of rollers 22', 22" is determined in such manner that during displacing of the second pair of rollers 22', 22" together with each particular section 1' of the absorbent band 1 as jammed there-between in a direction away from the first pair of rollers 21', 21", said absorbent band 1 is just partially torn, upon which said partially torn band 1 is introduced into said winding assembly 3, namely through the passage 31' in the rotor 31 as well as through the passage 32' in the stator 32 of each disposable winding unit 3', so that said band 1 is completely torn by means of rotating said rotor 32 of the winding assembly 3 during the reverse displacement of the second pair of rollers 22', 22" towards the first pair of rollers 21', 21".

In the apparatus as shown in Figs. 1 and 2, diameter of all rollers 21', 21"; 22', 22" is approximately 34 mm. The minimal distance between the rollers 22', 22" of the second pair in their first terminal position, and the rollers 21', 21" of the first pair is approximately 10 mm, and the distance between the rollers 22', 22" of the second pair in their second terminal position, and the rollers 21', 21" of the first pair is in this case approximately 20 mm. Displacing of the second pair of rollers 22', 22" in each corresponding rhythm is assured by means of optionally mechanical or pneumatic means, e.g. by at least one pneumatic cylinder, when desired.

In general, the circumferential velocity v₂ of rotating of the second pair of rollers 22', 22" in the opposite directions can be at least approximately equal to the circumferential velocity v₁ of rotating of the first pair of rollers 21', 21" in the opposite directions, by which each particular longitudinal section 1' of the absorbent band 1 is transferred towards the winding assembly 3 along a corresponding plane, and in the shown embodiments corresponds to rotating the rollers 21', 21" of the first pair by approx. 3,3 s⁻¹.

However, when desired, the circumferential velocity v₂ of rotating of the second pair of rollers 22', 22" in the opposite directions can also be some higher than the circumferential velocity v₁ of rotating of the first pair of rollers 21', 21" in the opposite directions, by which each particular longitudinal section 1' of the absorbent band 1 is transferred towards the winding assembly 3 along a corresponding plane. Within the context of the previously proposed diameter of the rollers 21', 21"; 22', 22" and by enabling oscillation of the second pair of rollers 22', 22" relative to the first pair of rollers 21', 21" at the distance between 10 and 20 mm, empirical experiences show that by velocity v₁ of rotating the first pair of rollers 21', 21" the velocity v₂ of rotating the second pair of rollers 22', 22" in the second pair can be adjusted to 3,3 to 5,5 s⁻¹. In such case the difference between said second circumferential velocity v₂ and said first circumferential velocity v₁ is such, that during reverse displacement of the second pair of rollers 22', 22" from its second terminal position towards its first terminal position adjacent to the first pair of rollers 21', 21" each potential formation of loops on the continuously between the first pair of rollers 21', 21" extending band 1 can be avoided, by which a fluent processing of said band 1 through both pairs of rollers 21', 21"; 22', 22" is assured without formation of loops or folding the band 1.

Regarding the shown embodiment, said tearing assembly 2 and said winding assembly 3 are adapted to cooperate with a further assembly 4, which is suitable for providing periodical initial weakening of the band 1 prior to its completely tearing-off, wherein said further assembly 4 is adjusted for providing of weak zones in the form of a plurality of mutually displaced notches or grooves or passages, which are arranged in a sequence, and with regard to the processing direction of the band 1 extend at least approximately in a transversal direction of the band 1. In this, said weakening assembly 4 for providing said periodical weakening of the band 1 prior to complete tearing-off comprises at least one apparatus, which is selected from devices, which are suitable for cutting, notching, punching, chemical etching, water jet cutting, air jet cutting, or cutting by laser beam.

Figs. 1 and 2 are moreover also suitable for schematic presentation of performing a method for manufacturing of a tampon by using an apparatus, which includes a tearing assembly 2 and a tearing assembly 3 in accordance with disclosed features.

Such method generally comprises
- providing of an endless band 1 consisting of absorbent fibers and having a pre-determined width and at least approximately uniform thickness;
- transferring said band 1 through an assembly 4, which is suitable for forming of along said band 1 periodically repeated weakening, which are formed either as cuttings or perforations or similar local weak zones, which are arranged in sequences extending at least approximately in the transversal direction of said band 1 and by which the longitudinal sections 1' of subsequently torn band 1 are defined;
- placing of a liquid permeable layer, namely a so called tampon wrapper, over said absorbent band 1, and placing of a retaining string around said absorbent band 1;
- transferring said absorbent band 1 through two pairs of oppositely rotating rollers 21', 21"; 22', 22" due to tearing of said band 1 and thus providing suitable longitudinal sections 1';
- transferring said band 1 towards the winding assembly 3, in which each longitudinal section 1' of the band 1 in each disposable winding unit 3' is inserted both through a passage 31' in the rotor 31 and the passage 32' in the stator 32, by which said rotor 31 is coaxially surrounded, upon which said rotor 31 is rotated and upon certain number of revolutions said section 1' of the band 1 is helically wound into at least approximately cylindrical blank, which us then followed by at least
- compression of said blank in its radial direction by means of a pre-determined number of jaws, which includes formation of a proximal area of the tampon into a shape, which corresponds either to digital or applicator tampon, so that said blank is transformed to a tampon having a pre-determined density of fibers and consequently also each corresponding absorptiveness and mechanical strength, and is moreover furnished with a plurality of longitudinally extending ribs together with grooves there-between, which are equidistantly spaced apart of each other in the circumferential direction of such obtained tampon.

In accordance with the present invention said method further provides that said absorbent band 1 is during its transfer throughout the tearing assembly 2 displaced by the first velocity v₁, which corresponds to unchangeable circumferential velocity by rotating of the first pair of rollers 21', 21", between which the band 1 is transferred, upon which said band 1 is transferred between the second pair of rollers 22', 22", the circumferential velocity v₂ of which is also unchangeable. In this, said first pair of rollers 21', 21" is maintained in its position, while the second pair of rollers 22', 22" is oscillated to and fro in a direction along the plane of transferring said band 1, namely from its first terminal position adjacent to the first pair of rollers 21', 21" to its second terminal position at a pre-determined distance apart from said first pair of rollers 21', 21", and vice versa, so that due to said oscillatory displacing of said second pair of rollers 22', 22" said absorbent band 1 is in the area between both pairs of rollers 21', 21"; 22', 22" just partially torn. Upon that, such partially torn band 1 is transferred towards each disposable winding unit 3' in the winding assembly 3, namely throughout the passage 31' in the rotor 31 and also throughout the passage 32' in the stator 32 surrounding said rotor 31, upon which after certain number of revolutions by rotating said rotor 31 and by simultaneously performing a reverse displacement of the second pair of rollers 22', 22" towards the first pair of rollers 21' 21" partially torn section 1' of said band 1 is completely torn-off and removed from the rest of the endless band 1.

In one of the variations of said method, the circumferential velocity v₂ of oppositely rotating second pair of rollers 22', 22" is maintained at approximately equal value as the circumferential velocity v₁ of the oppositely rotating first pair of rollers 21', 21", by which then also each longitudinal section 1' of the absorbent band 1 is forwarded towards the winding assembly 3 along the processing plane.

In an alternative variation of said method, the circumferential velocity v₂ of oppositely rotating second pair of rollers 22', 22" is maintained at higher value than the circumferential velocity v₁ of the oppositely rotating first pair of rollers 21', 21", by which each longitudinal section 1' of the absorbent band 1 is forwarded towards the winding assembly 3 along the processing plane, wherein the difference between said circumferential velocity v₂ of oppositely rotating second pair of rollers 22', 22" and the circumferential velocity v₁ of the oppositely rotating first pair of rollers 21', 21" is sufficient to prevent formation of a loop during the reverse movement of the second pair of rollers 22', 22" from its second terminal position towards its first terminal position adjacent to the first pair of rollers 21', 21" when the band 1 is continuously forwarded through the first pair of rollers 21', 21", by which a fluent forwarding of the band through both pair of rollers 21', 21"; 22', 22" is assured without forming any loops or folding of the band 1.

Said periodically arranged weak zones on the band 1 prior to final tearing are performed in the form of a sequence of cutoffs or notches or throughout passages, which are spaced apart from each other and arranged in a sequence extending at least approximately transversally with regard to a direction of forwarding said band 1, wherein said cutoffs or notches or throughout passages are made by cutting, notching, punching, chemical etching, water jet cutting, air jet cutting, or cutting by laser beam.

By performing said method all parameters may be used, which were previously disclosed in relationship with operation of the tearing assembly 2 and the winding assembly 3 in a tampon manufacturing apparatus.

## Claims

1. Tearing and winding assembly (2, 3) in a tampon manufacturing apparatus, especially for manufacturing of a hygienic tampon, consisting of an endless band (1), which is formed of absorbent fibers and cut to longitudinal sections (1') of a pre-determined length, and which is together with a liquid permeable wrapper and a retaining string helically wound into at least approximately cylindrical blank, which is then radially compressed into at least approximately cylindrical tampon, which is on its circumference furnished with a plurality of longitudinally extending ribs, which are along the circumference equidistantly spaced apart from each other, wherein said tearing assembly (2) in said tampon manufacturing apparatus comprises two pairs of guiding rollers (21', 21"; 22', 22"), wherein the rollers (21', 21") in the first pair are arranged at a pre-determined distance apart from each other, which is smaller than the thickness of said and (1), and are rotatably embedded in a casing, and are moreover pressed towards to each other with a pre-determined force and rotated in opposite directions with a pre-determined circumferential velocity (v₁) and are therefore suitable for guiding said band (1) there-between in a processing direction, namely in a direction towards the second pair of rollers (22', 22"), and wherein also the rollers (22', 22") in said second pair are arranged at a pre-determined distance apart from each other, which is smaller than thickness of said band (1), and are rotatably embedded in a suitable casing and are moreover also pressed towards to each other by a pre-determined force and simultaneously rotated in opposite directions with a circumferential velocity (v₂), and are therefore also suitable for guiding said band (1) there-between in a processing direction, namely towards a winding assembly (3) in a tampon manufacturing apparatus, wherein said winding assembly (3) comprises a pre-determined number of winding units (3'), which are arranged equidistantly along the circumference of a virtual cylinder, wherein each winding unit (3') comprises a bifurcated rotor (31), which is coaxially inserted into a tubular stator (32), wherein said rotor (31) is furnished with a diametrically extending throughout passage (31'), which is suitable for receiving said longitudinal section (1') of the band (1), and wherein also said stator (32) is furnished with a diametrically extending passage (32'), which is suitable for receiving of the same previously mentioned longitudinal section (1') of the band (1), so that consequently, as soon as each longitudinal section (1') of the band (1) is inserted both through said passage (32') in the stator (32) and said passage (31') in the rotor (31) and upon that by performing a pre-determined number of revolutions of the rotor (31), said winding unit (3) is suitable for formation of a helically wound section (1') of the absorbent band (1) together with said wrapper and said retaining string into an at least approximately cylindrical blank, which is ready to be radially compressed into a properly shaped and finalized tampon, and wherein in front of said tearing assembly (2) when taking in consideration said processing direction, a further assembly (4) is optionally available, which is intended for periodical weakening of the band (1) prior to tearing, by which the length of longitudinal sections (1') is defined, to which the band (1) is then cut, **characterized in that** said tearing assembly (2) comprises a stationary first pair of rollers (21', 21"), which are by circumferential velocity (v₁) rotated in opposite directions, as well as an oscillatory to and fro along the plane of transferring said absorbent band (1) displaceable second pair of rollers (22', 22"), which are also rotated in opposite directions by constant circumferential velocity (v₂), wherein by said oscillatory displacing said second pair of rollers (22', 22") is during uniformly and continuously transferring said band (1) between said pairs of rollers (21', 21"; 22', 22") by a velocity, which at least approximately corresponds to the circumferential velocity (v₁) of the first pair of rollers (21', 21"), in periodically repeating manner displaceable from its first terminal position, which is located adjacent to said first pair of rollers (21', 21"), to its second terminal position, which is located in said plane of transferring said sections of the band (1) at a pre-determined distance apart from said first pair of rollers (21', 21"), and vice versa, and wherein by oscillating said second pair of oppositely rotating rollers (22', 22") the circumferential velocity (v₂) of them remains unchangeable, while the distance between said first terminal position and said second terminal position of the second pair of rollers (22', 22") is determined in such manner that during displacing of the second pair of rollers (22', 22") together with each particular section (1') of the absorbent band (1) as jammed there-between in a direction away from the first pair of rollers (21', 21") said absorbent band (1) is just partially torn, upon which said partially torn band (1) is introduced into said winding assembly (3), namely through the passage (31') in the rotor (31) as well as through the passage (32') in the stator (32) of each disposable winding unit (3'), so that said band (1) is completely torn by means of rotating said rotor (32) of the winding assembly (3) during the reverse displacement of the second pair of rollers (22', 22") towards the first pair of rollers (21', 21").

2. Tearing and winding assembly (2, 3) according to Claim 1, **characterized in that** the circumferential velocity (v₂) of rotating of the second pair of rollers (22', 22") in the opposite directions at least approximately equals to the circumferential velocity (v₁) of rotating of the first pair of rollers (21', 21") in the opposite directions, by which each particular longitudinal section (1') of the absorbent band (1) is transferred towards the winding assembly (3) along a corresponding plane.

3. Tearing and winding assembly (2, 3) according to Claim 1, **characterized in that** the circumferential velocity (v₂) of rotating of the second pair of rollers (22', 22") in the opposite directions is higher than the circumferential velocity (v₁) of rotating of the first pair of rollers (21', 21") in the opposite directions, by which each particular longitudinal section (1') of the absorbent band (1) is transferred towards the winding assembly (3) along a corresponding plane, wherein the difference between said second circumferential velocity (v₂) and said first circumferential velocity (v₁) is such, that during reverse displacement of the second pair of rollers (22', 22") from its second terminal position towards its first terminal position adjacent to the first pair of rollers (21', 21") each potential formation of loops on the continuously between the first pair of rollers (21', 21") extending band (1) can be avoided, by which a fluent processing of said band (1) through both pairs of rollers (21', 21"; 22', 22") is assured without formation of loops or folding the band (1).

4. Tearing and winding assembly (2, 3) according to Claim 2 or 3, **characterized in that** said assemblies are adapted to cooperate with a further assembly (4), which is suitable for providing periodical initial weakening of the band (1) prior to its completely tearing-off, wherein said further assembly (4) is adjusted for providing of weak zones in the form of a plurality of mutually displaced notches or grooves or passages, which are arranged in a sequence, and with regard to the processing direction of the band (1) extend at least approximately in a transversal direction of the band (1).

5. Tearing and winding assembly (2, 3) according to Claim 4, **characterized in that** said assembly (4) for providing said periodical weakening of the band (1) prior to complete tearing-off comprises at least one apparatus, which is selected from devices, which are suitable for cutting, notching, punching, chemical etching, water jet cutting, air jet cutting, or cutting by laser beam.

6. Method for manufacturing of a tampon by using an apparatus comprising a tearing and winding assembly (2, 3) according to anyone of Claims 1 - 5, said method comprising
- providing of an endless band (1) consisting of absorbent fibers and having a pre-determined width and at least approximately uniform thickness;
- transferring said band (1) through an assembly (4), which is suitable for forming of along said band (1) periodically repeated weakening, which are formed either as cuttings or perforations or similar local weak zones, which are arranged in sequences extending at least approximately in the transversal direction of said band (1) and by which the longitudinal sections (1') of subsequently torn band (1) are defined;
- placing of a liquid permeable layer, namely a so called tampon wrapper, over said absorbent band (1), and placing of a retaining string around said absorbent band (1);
- transferring said absorbent band (1) through two pairs of oppositely rotating rollers (21', 21"; 22', 22") due to tearing of said band (1) and thus providing suitable longitudinal sections (1');
- transferring said band (1) towards the winding assembly (3), in which each longitudinal section (1') of the band (1) in each disposable winding unit (3') is inserted both through a passage (31') in the rotor (31) and the passage (32') in the stator (32), by which said rotor (31) is coaxially surrounded, upon which said rotor (31) is rotated and upon certain number of revolutions said section (1') of the band (1) is helically wound into at least approximately cylindrical blank, which us then followed by at least
- compression of said blank in its radial direction by means of a pre-determined number of jaws, which includes formation of a proximal area of the tampon into a shape, which corresponds either to digital or applicator tampon, so that said blank is transformed to a tampon having a pre-determined density of fibers and consequently also each corresponding absorptiveness and mechanical strength, and is moreover furnished with a plurality of longitudinally extending ribs together with grooves there-between, which are equidistantly spaced apart of each other in the circumferential direction of such obtained tampon,
**characterized in that**
said absorbent band (1) is during its transfer throughout the tearing assembly (2) displaced by the first velocity (v₁), which corresponds to unchangeable circumferential velocity by rotating of the first pair of rollers (21', 21"), between which the band (1) is transferred, upon which said band (1) is transferred between the second pair of rollers (22', 22"), the circumferential velocity (v₂) of which is also unchangeable, wherein the first pair of rollers (21', 21") is maintained in its position, while the second pair of rollers (22', 22") is oscillated to and fro in a direction along the plane of transferring said band (1), namely from its first terminal position adjacent to the first pair of rollers (21', 21") to its second terminal position at a pre-determined distance apart from said first pair of rollers (21', 21"), and vice versa, so that due to said oscillatory displacing of said second pair of rollers (22', 22") said absorbent band (1) is in the area between both pairs of rollers (21', 21"; 22', 22") just partially torn, upon which such partially torn band (1) is transferred towards each disposable winding unit (3') in the winding assembly (3), namely throughout the passage (31') in the rotor (31) and also throughout the passage (32') in the stator (32) surrounding said rotor (31), upon which after certain number of revolutions by rotating said rotor (31) and by simultaneously performing a reverse displacement of the second pair of rollers (22', 22") towards the first pair of rollers (21' 21") each partially torn section (1') of said band (1) is completely torn and separated from the rest of said endless band (1).

7. Method according to Claim 6, **characterized in that** the circumferential velocity (v₂) of oppositely rotating second pair of rollers (22', 22") is maintained at approximately equal value as the circumferential velocity (v₁) of the oppositely rotating first pair of rollers (21', 21"), by which then also each longitudinal section (1') of the absorbent band (1) is forwarded towards the winding assembly (3) along the processing plane.

8. Method according to Claim 6, **characterized in that** the circumferential velocity (v₂) of oppositely rotating second pair of rollers (22', 22") is maintained at higher value than the circumferential velocity (v₁) of the oppositely rotating first pair of rollers (21', 21"), by which each longitudinal section (1') of the absorbent band (1) is forwarded towards the winding assembly (3) along the processing plane, wherein the difference between said circumferential velocity (v₂) of oppositely rotating second pair of rollers (22', 22") and the circumferential velocity (v₁) of the oppositely rotating first pair of rollers (21', 21") is sufficient to prevent formation of a loop during the reverse movement of the second pair of rollers (22', 22") from its second terminal position towards its first terminal position adjacent to the first pair of rollers (21', 21") when the band (1) is continuously forwarded through the first pair of rollers (21', 21"), by which a fluent forwarding of band through both pair of rollers (21', 21"; 22', 22") is assured without forming any loops or folding of the band (1).

9. Method according to Claim 7 or 8, **characterized in that** said periodically arranged weak zones on the band (1) prior to final tearing are performed in the form of a sequence of cutoffs or notches or throughout passages, which are spaced apart from each other and arranged in a sequence extending at least approximately transversally with regard to a direction of forwarding said band (1), wherein said cutoffs or notches or throughout passages are made by cutting, notching, punching, chemical etching, water jet cutting, air jet cutting, or cutting by laser beam.

## Patentansprüche

1. Abreiß- und Aufwickelvorrichtung (2, 3) in einer Tamponherstellungsvorrichtung, insbesondere zur Herstellung eines Hygienetampons, bestehend aus einem Endlosband (1), das aus saugfähigen Fasern gebildet und in Längsabschnitte (1') einer vorbestimmten Länge geschnitten ist, und das zusammen mit einer flüssigkeitsdurchlässigen Hülle und einer Halteschnur spiralförmig zu einem zumindest annähernd zylindrischen Rohling gewickelt ist, der anschließend radial zu einem zumindest annähernd zylindrischen Tampon verdichtet wird, der an seinem Umfang mit einer Vielzahl von sich in Längsrichtung erstreckenden Rippen versehen ist, die entlang des Umfangs in gleichen Abständen voneinander beabstandet sind, wobei die Abreißvorrichtung (2) in der Tamponherstellungsvorrichtung zwei Paare von Führungsrollen (21', 21"; 22', 22") umfasst, wobei die Rollen (21', 21") in dem ersten Paar in einem vorbestimmten Abstand voneinander angeordnet sind, der kleiner ist als die Dicke des Bandes (1), und drehbar in einem Gehäuse eingebettet sind, und außerdem mit einer vorbestimmten Kraft gegeneinander gedrückt und mit einer vorbestimmten Umfangsgeschwindigkeit (v₁) in entgegengesetzte Richtungen gedreht werden und daher geeignet sind, das Band (1) zwischen sich in einer Verarbeitungsrichtung, nämlich in einer Richtung hin zum zweiten Rollenpaar (22', 22"), zu führen, und wobei auch die Rollen (22', 22") in dem zweiten Paar in einem vorbestimmten Abstand voneinander angeordnet sind, der kleiner ist als die Dicke des Bandes (1), und drehbar in einem geeigneten Gehäuse eingebettet sind und außerdem mit einer vorbestimmten Kraft gegeneinander gedrückt und gleichzeitig mit einer Umfangsgeschwindigkeit (v₂) in gegenläufige Richtungen gedreht werden, und daher auch geeignet sind, das Band (1) zwischen sich in einer Bearbeitungsrichtung, nämlich zu einer Aufwickelvorrichtung (3) in einer Tamponherstellungsvorrichtung, zu führen, wobei die Aufwickelvorrichtung (3) eine vorbestimmte Anzahl von Aufwickeleinheiten (3') umfasst, die in gleichen Abständen entlang des Umfangs eines virtuellen Zylinders angeordnet sind, wobei jede Aufwickeleinheit (3') einen gabelförmigen Rotor (31) umfasst, der koaxial in einen rohrförmigen Stator (32) eingesetzt ist, wobei der Rotor (31) mit einem diametral verlaufenden durchgehenden Durchgang (31') versehen ist, der zur Aufnahme des Längsabschnitts (1') des Bandes (1) geeignet ist, und wobei auch der Stator (32) mit einem diametral verlaufenden Durchgang (32') versehen ist, der zur Aufnahme des zuvor genannten Längsabschnitts (1') des Bandes (1) geeignet ist, so dass folglich, sobald jeder Längsabschnitt (1') des Bandes (1) sowohl durch den Durchgang (32') im Stator (32) als auch durch den Durchgang (31') im Rotor (31) eingeführt ist und daraufhin durch Ausführen einer vorbestimmten Anzahl von Umdrehungen des Rotors (31), die Aufwickeleinheit (3) geeignet ist, einen spiralförmig gewickelten Abschnitt (1') des saugfähigen Bandes (1) zusammen mit der Hülle und der Halteschnur zu einem zumindest annähernd zylindrischen Rohling zu formen, der bereit ist, radial zu einem richtig geformten und fertigen Tampon zusammengedrückt zu werden, und wobei vor der Abreißvorrichtung (2), unter Berücksichtigung der Bearbeitungsrichtung, optional eine weitere Vorrichtung (4) vorgesehen ist, die dazu bestimmt ist, das Band (1) vor dem Abreißen periodisch zu schwächen, wodurch die Länge der Längsabschnitte (1') definiert wird, in die das Band (1) dann geschnitten wird, **dadurch gekennzeichnet, dass** die Abreißvorrichtung (2) ein stationäres erstes Rollenpaar (21', 21") umfasst, die durch die Umfangsgeschwindigkeit (v₁) in gegenläufige Richtungen gedreht werden, sowie ein oszillierend entlang der Übertragungsebene des saugfähigen Bandes (1) hin und her verschiebbares zweites Rollenpaar (22', 22") aufweist, das ebenfalls mit konstanter Umfangsgeschwindigkeit (v₂) in gegenläufigen Richtungen gedreht wird, wobei durch die oszillierende Verschiebung da zweite Rollenpaar (22', 22"), bei gleichmäßiger und kontinuierlicher Übertragung des Bandes (1) zwischen den Rollenpaaren (21', 21"; 22', 22") mit einer Geschwindigkeit, die zumindest annähernd der Umfangsgeschwindigkeit (v₁) des ersten Rollenpaares (21', 21") entspricht, in periodisch wiederholbarer Weise aus seiner ersten Endposition, die benachbart zu dem ersten Rollenpaar (21', 21") angeordnet ist, in seine zweite Endposition verschiebbar ist, die in der Ebene der Übertragung der Abschnitte des Bandes (1) in einem vorbestimmten Abstand von dem ersten Rollenpaar (21', 21") angeordnet ist, und umgekehrt, und wobei durch Oszillieren des zweiten Paares von gegenläufig rotierenden Rollen (22', 22") die Umfangsgeschwindigkeit (v₂) dieser Rollen unveränderlich bleibt, während der Abstand zwischen der ersten Endposition und der zweiten Endposition des zweiten Rollenpaares (22', 22") so festgelegt ist, dass beim Verschieben des zweiten Rollenpaares (22', 22") zusammen mit jedem einzelnen Abschnitt (1') des dazwischen eingeklemmten saugfähigen Bandes (1) in einer Richtung weg vom ersten Paar von Rollen (21', 21") das saugfähige Band (1) gerade teilweise abreißt, woraufhin das teilweise abgerissene Band (1) in die Aufwickelvorrichtung (3) eingeführt wird, und zwar durch den Durchgang (31') im Rotor (31) sowie durch den Durchgang (32') im Stator (32) jeder verfügbaren Aufwickeleinheit (3'), so dass das Band (1) durch Drehen des Rotors (32) der Aufwickelvorrichtung (3) während der Rückwärtsbewegung des zweiten Rollenpaares (22', 22") in Richtung zum ersten Rollenpaar (21', 21") vollständig abreißt.

2. Abreiß- und Aufwickelvorrichtung (2, 3) nach Anspruch 1, **dadurch gekennzeichnet, dass** die Umfangsgeschwindigkeit (v₂) der Drehung des zweiten Rollenpaares (22', 22") in gegenläufigen Richtungen mindestens annähernd gleich der Umfangsgeschwindigkeit (v₁) der Drehung des ersten Rollenpaares (21', 21") in gegenläufigen Richtungen ist, wodurch jeder einzelne Längsabschnitt (1') des saugfähigen Bandes (1) entlang einer entsprechenden Ebene zur Aufwickelvorrichtung (3) transportiert wird.

3. Abreiß- und Aufwickelvorrichtung (2, 3) nach Anspruch 1, **dadurch gekennzeichnet, dass** die Umfangsgeschwindigkeit (v₂) der Drehung des zweiten Rollenpaares (22', 22") in gegenläufige Richtungen höher ist als die Umfangsgeschwindigkeit (v₁) der Drehung des ersten Rollenpaares (21', 21") in gegenläufige Richtungen, wodurch jeder einzelne Längsabschnitt (1') des saugfähigen Bandes (1) entlang einer entsprechenden Ebene zur Aufwickelvorrichtung (3) transportiert wird, wobei die Differenz zwischen der zweiten Umfangsgeschwindigkeit (v₂) und der ersten Umfangsgeschwindigkeit (v₁) so ist, dass während der Rückwärtsbewegung des zweiten Rollenpaares (22', 22") aus seiner zweiten Endposition zu seiner ersten Endposition neben dem ersten Rollenpaar (21', 21") jede mögliche Bildung von Schlaufen an dem sich kontinuierlich zwischen dem ersten Rollenpaar (21', 21") erstreckenden Band (1) vermieden werden kann, wodurch eine flüssige Verarbeitung des Bandes (1) durch beide Rollenpaare (21', 21"; 22', 22") ohne Bildung von Schlaufen oder Falten des Bandes (1) gewährleistet ist.

4. Abreiß- und Aufwickelvorrichtung (2, 3) nach Anspruch 2 oder 3, **dadurch gekennzeichnet, dass** die Vorrichtungen so ausgelegt sind, dass sie mit einer weiteren Vorrichtung (4) zusammenwirken, die dazu geeignet ist, das Band (1) vor seinem vollständigen Abreißen periodisch anfänglich zu schwächen, wobei die weitere Vorrichtung (4) dazu ausgelegt ist, Schwächungszonen in Form einer Vielzahl von gegenseitig versetzten Kerben oder Nuten oder Durchgängen zu bilden, die in einer Folge angeordnet sind und sich in Bezug auf die Verarbeitungsrichtung des Bandes (1) zumindest annähernd in einer Querrichtung des Bandes (1) erstrecken.

5. Abreiß- und Aufwickelvorrichtung (2, 3) nach Anspruch 4, **dadurch gekennzeichnet, dass** die Anordnung (4) zum periodischen Schwächen des Bandes (1) vor seinem vollständigen Abreißen mindestens eine Vorrichtung umfasst, die aus Vorrichtungen ausgewählt ist, die zum Schneiden, Einkerben, Stanzen, chemischen Ätzen, Wasserstrahlschneiden, Luftstrahlschneiden oder Schneiden mittels Laserstrahl geeignet sind.

6. Verfahren zur Herstellung eines Tampons unter Verwendung einer Vorrichtung mit einer Abreiß- und Aufwickelvorrichtung (2, 3) gemäß einem der Ansprüche 1 bis 5, wobei das Verfahren umfasst:
- Bereitstellen eines Endlosbandes (1), das aus saugfähigen Fasern besteht und eine vorbestimmte Breite und mindestens annähernd gleichmäßige Dicke aufweist;
- Transportieren des Bandes (1) durch eine Vorrichtung (4), die geeignet ist, entlang des Bandes (1) periodisch wiederholte Schwächungen zu bilden, die entweder als Einschnitte oder Perforationen oder ähnliche lokale Schwachstellen ausgebildet sind, die in Reihen angeordnet sind, die sich zumindest annähernd in Querrichtung des Bandes (1) erstrecken und durch die die Längsschnitte (1') des anschließend abgerissenen Bandes (1) definiert sind;
- Anbringen einer flüssigkeitsdurchlässigen Schicht, nämlich einer sogenannten Tamponhülle, über dem saugfähigen Band (1) und Anbringen einer Halteschnur um das saugfähige Band (1);
- Transportieren des saugfähigen Bandes (1) durch zwei Paare gegenläufig rotierender Rollen (21', 21"; 22', 22") aufgrund des Zerreißens des Bandes (1) und dadurch Bereitstellen geeigneter Längsabschnitte (1');
- Transportieren des Bandes (1) zur Aufwickelvorrichtung (3), in der jeder Längsabschnitt (1') des Bandes (1) in jeder verfügbaren Aufwickeleinheit (3') sowohl durch einen Durchgang (31') im Rotor (31) als auch durch den Durchgang (32') im Stator (32), von dem der Rotor (31) koaxial umgeben ist, eingeführt wird, woraufhin der Rotor (31) gedreht wird und nach einer bestimmten Anzahl von Umdrehungen der Abschnitt (1') des Bandes (1) spiralförmig zu einem zumindest annähernd zylindrischen Rohling aufgewickelt wird, woraufhin mindestens Folgendes folgt:
- Kompression des Rohlings in seiner Radialrichtung mittels einer vorbestimmten Anzahl von Backen, was das Bilden eines proximalen Bereichs des Tampons in eine Form beinhaltet, die entweder einem Finger- oder einem Applikator-Tampon entspricht, so dass der Rohling in einen Tampon mit einer vorbestimmten Faserdichte und folglich auch mit einer entsprechenden Saugfähigkeit und mechanischen Festigkeit umgeformt wird und darüber hinaus mit einer Vielzahl von sich in Längsrichtung erstreckenden Rippen und dazwischen liegenden Nuten versehen wird, die in Umfangsrichtung des so erhaltenen Tampons in gleichen Abständen voneinander angeordnet sind,
**dadurch gekennzeichnet, dass**
das saugfähige Band (1) während seines Transports durch die Abreißvorrichtung (2) mit der ersten Geschwindigkeit (v₁) verschoben wird, die der unveränderlichen Umfangsgeschwindigkeit durch die Drehung des ersten Rollenpaares (21', 21") entspricht, zwischen denen das Band (1) transportiert wird, woraufhin das Band (1) zwischen dem zweiten Rollenpaar (22', 22") transportiert wird, deren Umfangsgeschwindigkeit (v₂) ebenfalls unveränderlich ist, wobei das erste Rollenpaar (21', 21") in seiner Position gehalten wird, während das zweite Rollenpaar (22', 22") in einer Richtung entlang der Ebene der Übertragung des Bandes (1) hin und her oszilliert wird, und zwar von seiner ersten Endposition neben dem ersten Rollenpaar (21', 21") zu seiner zweiten Endposition in einem vorbestimmten Abstand von dem ersten Rollenpaar (21', 21"), und umgekehrt, so dass aufgrund der oszillierenden Verschiebung des zweiten Rollenpaares (22', 22") das saugfähige Band (1) in dem Bereich zwischen beiden Rollenpaaren (21', 21"; 22', 22") nur teilweise abreißt, woraufhin das teilweise abgerissene Band (1) zu jeder verfügbaren Aufwickeleinheit (3') in der Aufwickelvorrichtung (3) transportiert wird, und zwar durch den Durchgang (31') im Rotor (31) und auch durch den Durchgang (32') im Stator (32), der den Rotor (31) umgibt, worauf nach einer bestimmten Anzahl von Umdrehungen durch Drehen des Rotors (31) und durch gleichzeitiges Ausführen einer Rückwärtsbewegung des zweiten Rollenpaares (22', 22") hin zum ersten Rollenpaar (21', 21") jeder teilweise abgerissene Abschnitt (1') des Bandes (1) vollständig abgerissen und vom Rest des Endlosbandes (1) getrennt wird.

7. Verfahren nach Anspruch 6, **dadurch gekennzeichnet, dass** die Umfangsgeschwindigkeit (v₂) des entgegengesetzt rotierenden zweiten Rollenpaares (22', 22") auf ungefähr dem gleichen Wert wie die Umfangsgeschwindigkeit (v₁) des entgegengesetzt rotierenden ersten Rollenpaares (21', 21") gehalten wird, wodurch dann auch jeder Längsabschnitt (1') des saugfähigen Bandes (1) entlang der Bearbeitungsebene zur Aufwickelvorrichtung (3) weiterbefördert wird.

8. Verfahren nach Anspruch 6, **dadurch gekennzeichnet, dass** die Umfangsgeschwindigkeit (v₂) des gegenläufig rotierenden zweiten Rollenpaares (22', 22") auf einem höheren Wert gehalten wird als die Umfangsgeschwindigkeit (v₁) des gegenläufig rotierenden ersten Rollenpaares (21', 21"), wodurch jeder Längsabschnitt (1') des saugfähigen Bandes (1) entlang der Bearbeitungsebene zur Aufwickelvorrichtung (3) weiterbefördert wird, wobei die Differenz zwischen der Umfangsgeschwindigkeit (v₂) des gegenläufig rotierenden zweiten Rollenpaares (22', 22") und der Umfangsgeschwindigkeit (v₁) des entgegengesetzt rotierenden ersten Rollenpaares (21', 21") ausreichend ist, um die Schlaufenbildung während der Rückwärtsbewegung des zweiten Rollenpaares (22', 22") aus seiner zweiten Endposition zu seiner ersten Endposition neben dem ersten Rollenpaar (21', 21") zu verhindern, wenn das Band (1) kontinuierlich durch das erste Rollenpaar (21', 21") weiterbefördert wird, wodurch eine flüssige Weiterbeförderung des Bandes durch beide Rollenpaare (21', 21"; 22', 22") ohne Bildung von Schlaufen oder Falten des Bandes (1) gewährleistet ist.

9. Verfahren nach Anspruch 7 oder 8, **dadurch gekennzeichnet, dass** die periodisch angeordneten Schwachstellen auf dem Band (1) vor dem endgültigen Abreißen in Form einer Folge von Ausschnitten oder Kerben oder Durchbrüchen ausgebildet sind, die voneinander beabstandet und in einer Folge angeordnet sind, die sich zumindest annähernd quer zu einer Förderrichtung des Bandes (1) erstreckt, wobei die Abschnitte oder Kerben oder Durchgänge durch Schneiden, Einkerben, Stanzen, chemisches Ätzen, Wasserstrahlschneiden, Luftstrahlschneiden oder Schneiden mittels Laserstrahl hergestellt werden.

## Revendications

1. Ensemble de déchirement et d'enroulement (2,3) dans un appareil de fabrication de tampon, en particulier afin de fabriquer un tampon hygiénique, constitué d'une bande sans fin (1), qui est constituée de fibres absorbantes et coupées selon des sections longitudinales (1') d'une longueur prédéterminée, et qui est enroulée de manière hélicoïdale avec un papier perméable au liquide et une ficelle de retenue dans au moins une ébauche approximativement cylindrique, qui est ensuite comprimée de manière radiale dans au moins un tampon approximativement cylindrique, qui est, sur sa circonférence, doté d'une pluralité de nervures s'étendant de manière longitudinale, qui sont espacées le long de la circonférence à équidistance l'une de l'autre, dans lequel ledit ensemble de déchirement (2) dans ledit appareil de fabrication de tampon comprend deux paires de galets de guidage (21', 21" ; 22', 22"), dans lequel les galets (21', 21") dans la première paire sont agencés à une distance prédéterminée, éloignés l'un de l'autre, qui est inférieure à l'épaisseur de ladite bande (1), et sont intégrés en rotation dans un boîtier, et sont en outre comprimés l'un vers l'autre avec une force prédéterminée et tournés dans des directions opposées avec une vitesse circonférentielle prédéterminée (v₁) et sont donc adaptés afin de guider ladite bande (1) entre eux dans une direction de traitement, à savoir dans une direction vers la seconde paire de galets (22', 22") et dans lequel également les galets (22', 22") dans ladite seconde paire sont agencés à une distance prédéterminée, éloignés l'un de l'autre, qui est inférieure à l'épaisseur de ladite bande (1), et sont intégrés en rotation dans un boîtier adapté et sont en outre comprimés l'un vers l'autre par une force prédéterminée et simultanément tournés dans des directions opposées avec une vitesse circonférentielle (v₂), et sont donc également adaptés afin de guider ladite bande (1) entre eux dans une direction de traitement, à savoir vers un ensemble d'enroulement (3) dans un appareil de fabrication de tampon, dans lequel ledit ensemble d'enroulement (3) comprend un nombre prédéterminé d'unités d'enroulement (3'), qui sont agencées à équidistance le long de la circonférence d'un cylindre virtuel, dans lequel chaque unité d'enroulement (3') comprend un rotor divisé (31), qui est inséré de manière coaxiale dans un stator tubulaire (32), dans lequel ledit rotor (31) est doté d'un passage traversant s'étendant diamétralement (31'), qui est adapté à recevoir ladite section longitudinale (1') de la bande (1), et dans lequel également ledit stator (32) est doté d'un passage s'étendant diamétralement (32'), qui est apte à recevoir la même section longitudinale mentionnée précédemment (1') de la bande (1), de sorte que, par conséquent, dès que chaque section longitudinale (1') de la bande (1) est insérée à la fois à travers ledit passage (32') dans le stator (32) et ledit passage (31') dans le rotor (31) et ainsi, en réalisant un nombre prédéterminé de tours du rotor (31), ladite unité d'enroulement (3) est apte à former une section enroulée hélicoïdale (1') de la bande absorbante (1) avec ledit papier et ladite ficelle de retenue dans une ébauche au moins approximativement cylindrique, qui est prête à être comprimée de manière radiale en un tampon finalisé et de forme correcte,, et dans lequel face audit ensemble de déchirement (2) lorsque l'on tient compte de ladite direction de traitement, un autre ensemble (4) est optionnellement disponible, qui est destiné à affaiblir régulièrement la bande (1) avant le déchirement, de sorte que la longueur des sections longitudinales (1') est définie, à laquelle la bande (1) est ensuite coupée, **caractérisé en ce que** ledit ensemble de déchirement (2) comprend une première paire fixe de galets (21', 21"), qui sont tournés à une vitesse circonférentielle (v₁) dans des directions opposées, ainsi qu'une seconde paire de galets (22', 22") pouvant être déplacés en va et vient oscillatoire le long du plan de transfert de ladite bande absorbante (1), qui sont également tournés dans des directions opposées à une vitesse circonférentielle constante (v₂), dans lequel pour ledit déplacement oscillatoire, ladite seconde paire de galets (22', 22") pendant un transfert uniforme et continu de ladite bande (1) entre lesdites paires de galets (21', 21" ; 22', 22") à une vitesse, qui correspond au moins approximativement à la vitesse circonférentielle (v₁) de la première paire de galets (21', 21"), est de manière répétée régulière déplaçable de leur première position terminale, qui est située près de ladite première paire de galets (21', 21"), à leur seconde position terminale, qui est située dans ledit plan de transfert desdites sections de la bande (1) à une distance prédéterminée éloignée de ladite première paire de galets (21', 21"), et inversement, et dans lequel, en faisant osciller ladite seconde paire de galets tournant dans un sens opposé (22', 22"), leur vitesse circonférentielle (v₂) reste inchangée, tandis que la distance entre ladite première position terminale et ladite seconde position terminale de la seconde paire de galets (22', 22") est déterminée de telle sorte que, pendant le déplacement de la seconde paire de galets (22', 22") avec chaque section particulière (1') de la bande absorbante (1) comme prise entre eux dans une direction s'éloignant de la première paire de galets (21', 21"), ladite bande absorbante (1) est juste partiellement déchirée, et ladite bande partiellement déchirée (1) est introduite dans ledit ensemble d'enroulement (3), à savoir à travers le passage (31') dans le rotor (31) ainsi qu'à travers le passage (32') dans le stator (32) de chaque unité d'enroulement disponible (3'), de sorte que ladite bande (1) soit totalement déchirée au moyen de la rotation dudit rotor (32) de l'ensemble d'enroulement (3) pendant le déplacement en sens inverse de la seconde paire de galets (22', 22") vers la première paire de galets (21', 21").

2. Ensemble de déchirement et d'enroulement (2, 3) selon la revendication 1, **caractérisé en ce que** la vitesse circonférentielle (v₂) de rotation de la seconde paire de galets (22', 22") dans les directions opposées est au moins approximativement égale à la vitesse circonférentielle (v₁) de rotation de la première paire de galets (21', 21") dans les directions opposées, de sorte que chaque section longitudinale particulière (1') de la bande absorbante (1) est transférée vers l'ensemble d'enroulement (3) le long d'un plan correspondant.

3. Ensemble de déchirement et d'enroulement (2, 3) selon la revendication 1, **caractérisé en ce que** la vitesse circonférentielle (v₂) de rotation de la seconde paire de galets (22', 22") dans les directions opposées est supérieure à la vitesse circonférentielle (v₁) de rotation de la première paire de galets (21', 21") dans les directions opposées, de sorte que chaque section longitudinale particulière (1') de la bande absorbante (1) est transférée vers l'ensemble d'enroulement (3) le long d'un plan correspondant, dans lequel la différence entre ladite seconde vitesse circonférentielle (v₂) et ladite première vitesse circonférentielle (v₁) est telle que pendant le déplacement en sens inverse de la seconde paire de galets (22', 22") de leur seconde position terminale vers leur première position terminale adjacente à la première paire de galets (21', 21"), chaque formation potentielle de boucles sur la bande (1) s'étendant en continu entre la première paire de galets (21', 21") peut être évitée, de sorte qu'un traitement fluide de ladite bande (1) à travers les deux paires de galets (21', 21" ; 22', 22") est assuré sans formation de boucles ou sans plier la bande (1).

4. Ensemble de déchirement et d'enroulement (2, 3) selon la revendication 2 ou 3, **caractérisé en ce que** lesdits ensembles sont aptes à coopérer avec un autre ensemble (4), qui est adapté afin de fournir un affaiblissement initial régulier de la bande (1) avant son déchirement total, dans lequel ledit autre ensemble (4) est ajusté afin de fournir des zones faibles sous la forme d'une pluralité d'encoches ou de rainures ou de passages réciproquement déplacés, qui sont agencés en séquence, et qui, par rapport à la direction de traitement de la bande (1), s'étendent au moins approximativement dans une direction transversale de la bande (1).

5. Ensemble de déchirement et d'enroulement (2, 3) selon la revendication 4, **caractérisé en ce que** ledit ensemble (4) permettant de fournir ledit affaiblissement régulier de la bande (1) avant le déchirement complet comprend au moins un appareil, qui est sélectionné parmi des dispositifs, qui sont adaptés afin de couper, de cranter, de poinçonner, de graver chimiquement, de couper au jet d'eau, de couper au jet d'air, ou de couper par un faisceau laser.

6. Procédé de fabrication d'un tampon en utilisant un appareil comprenant un ensemble de déchirement et d'enroulement (2, 3) selon l'une quelconque des revendications 1 à 5, ledit procédé comprenant :
- la fourniture d'une bande sans fin (1) constituée de fibres absorbantes et présentant une largeur prédéterminée et une épaisseur au moins approximativement uniforme ;
- le transfert de ladite bande (1) à travers un ensemble (4), qui est adapté afin de former le long de ladite bande (1) des zones d'affaiblissement régulièrement répétées, qui sont formées soit par le biais de coupes ou de perforations ou de zones faibles locales similaires, qui sont agencées dans des séquences s'étendant au moins approximativement dans la direction transversale de ladite bande (1) et par lesquelles les sections longitudinales (1') de la bande déchirée par la suite (1) sont définies ;
- le placement d'une couche perméable au liquide, à savoir un papier d'emballage de tampon, sur ladite bande absorbante (1), et le placement d'une ficelle de retenue autour de ladite bande absorbante (1) ;
- le transfert de ladite bande absorbante (1) à travers deux paires de galets tournant dans un sens opposé (21', 21" ; 22', 22") du fait du déchirement de ladite bande (1) et la fourniture ainsi de sections longitudinales adaptées (1') ;
- le transfert de ladite bande (1) vers l'ensemble d'enroulement (3), dans lequel chaque section longitudinale (1') de la bande (1) dans chaque unité d'enroulement disponible (3') est insérée à la fois à travers un passage (31') dans le rotor (31) et le passage (32') dans le stator (32), par lequel ledit rotor (31) est entouré de manière coaxiale, après quoi ledit rotor (31) est tourné et lors de certains tours, ladite section (1') de la bande (1) est enroulée de manière hélicoïdale dans au moins une ébauche approximativement cylindrique, qui est suivie d'au moins :
- une compression de ladite ébauche dans sa direction radiale au moyen d'un nombre de mâchoires prédéterminé, qui comprend la formation d'une zone proximale du tampon dans une forme, qui correspond soit à un tampon digital, soit à un tampon à applicateur, de sorte que ladite ébauche se transforme en un tampon présentant une densité de fibres prédéterminée et par conséquent également chaque capacité d'absorption et force mécanique correspondante, et est en outre doté d'une pluralité de nervures s'étendant de manière longitudinale ensemble avec des rainures entre elles, qui sont espacées les unes des autres de manière équidistante dans la direction circonférentielle de ce tampon obtenu,
**caractérisé en ce que**
ladite bande absorbante (1) est, pendant son transfert à travers l'ensemble de déchirement (2) déplacée à la première vitesse (v₁), qui correspond à une vitesse circonférentielle inchangée en tournant la première paire de galets (21', 21"), entre lesquels la bande (1) est transférée, après quoi ladite bande (1) est transférée entre la seconde paire de galets (22', 22"), à la vitesse circonférentielle (v₂) également inchangée, dans lequel la première paire de galets (21', 21") est maintenue dans sa position, tandis que la seconde paire de galets (22', 22") oscille en va et vient dans une direction le long du plan de transfert de ladite bande (1), à savoir de sa première position terminale qui est près de ladite première paire de galets (21', 21") à sa deuxième position terminale à une distance prédéterminée à l'écart de ladite première paire de galets (21', 21"), et inversement, de sorte que du fait du déplacement oscillatoire de ladite seconde paire de galets (22', 22"), ladite bande absorbante (1) se trouve dans la zone entre les deux paires de galets (21', 21" ; 22', 22") juste partiellement déchirée, après quoi cette bande partiellement déchirée (1) est transférée vers chaque unité d'enroulement disponible (3') dans l'ensemble d'enroulement (3), en particulier à travers le passage (31') dans le rotor (31) et également à travers le passage (32') dans le stator (32) entourant ledit rotor (31), de sorte que après un certain nombre de tours en tournant ledit rotor (31) et en réalisant simultanément un déplacement inverse de la seconde paire de galets (22', 22") vers la première paire de galets (21', 21"), chaque section partiellement déchirée (1') de ladite bande (1) est totalement déchirée et séparée du reste de ladite bande sans fin (1).

7. Procédé selon la revendication 6, **caractérisé en ce que** la vitesse circonférentielle (v₂) de la seconde paire de galets (22', 22") en rotation opposée est maintenue à une valeur approximativement égale à la vitesse circonférentielle (v₁) de la première paire de galets (21', 21"), en rotation opposée, de sorte que ainsi chaque section longitudinale (1') de la bande absorbante (1) est également transférée vers l'ensemble d'enroulement (3) le long du plan de traitement.

8. Procédé selon la revendication 6, **caractérisé en ce que** la vitesse circonférentielle (v₂) de la seconde paire de galets (22', 22") en rotation opposée est maintenue à une valeur supérieure à la vitesse circonférentielle (v₁) de la première paire de galets (21', 21"), en rotation opposée, de sorte que chaque section longitudinale (1') de la bande absorbante (1) est transférée vers l'ensemble d'enroulement (3) le long du plan de traitement, dans lequel la différence entre ladite vitesse circonférentielle (v₂) de la seconde paire de galets (22', 22") à rotation opposée et la vitesse circonférentielle (v₁) de la première paire de galets à rotation opposée (21', 21") est suffisante afin de prévenir la formation d'une boucle pendant le mouvement inverse de la seconde paire de galets (22', 22") de sa seconde position terminale vers sa première position terminale adjacente à la première paire de galets (21', 21"), quand la bande (1) est transférée de manière continue à travers la première paire de galets (21', 21"), de sorte qu'un transfert fluide de la bande à travers les deux paires de galets (21', 21" ; 22', 22") est assuré sans former de boucles ou sans plier la bande (1).

9. Procédé selon la revendication 7 ou 8, **caractérisé en ce que** lesdites zones faibles agencées de manière régulière sur la bande (1) avant le déchirement final sont réalisées sous la forme d'une séquence de découpes ou d'encoches ou de passages traversants, qui sont espacés les uns des autres et agencés en séquence s'étendant au moins approximativement de manière transversale eu égard à une direction de transfert de ladite bande (1), dans lequel lesdites découpes ou encoches ou passages traversants sont réalisées par découpe, crantage, poinçonnage, gravure chimique, coupure à jet d'eau, coupure à jet d'air, ou coupure par faisceau laser.
